# EUROPEAN PATENT APPLICATION

(11) **EP 1 142 583 A1**
(43) Date of publication of application: **10.10.2001**
(21) Application number: 00900040.7
(22) Date of filing: 04.01.2000
(51) Int. Cl.: A61K 45/00, A61K 38/08, A61K 35/00

(54) **ANTITUMOR AGENTS**

(30) Priority: 08.01.1999 JP 297199
(71) Applicant: TEIKOKU HORMONE MFG. CO., LTD., Tokyo 107-8522 (JP)
(72) Inventor: KOHNO, Michiaki, Nagasaki 852-8013 (JP); WATANABE, Kazushi, Kawasaki-shi, Kanagawa 211-0053 (JP)
(74) Representative: Weisert, Annekäte, Dipl.-Ing. Dr.-Ing.
(86) International application number: JP0000002
(87) International publication number: WO0040268

(57) **Abstract**

When a microtubule-interfering agent having an antitumor effect is used in combination with an ERK-MAP kinase cascade inhibitor, the antitumor effect of the microtubule-interfering agent is remarkably potentiated. Accordingly, a combination of a microtubule-interfering agent and an ERK-MAP kinase cascade inhibitor is useful as a very effective antitumor agent.

## Description

### Technical Field

This invention relates to the use of a combination of a microtubule-interfering agent and an ERK-MAP kinase cascade inhibitor as an antitumor agent.

### Background Art

Microtubules are tubular protein fibers having a diameter of about 25 nm and occurring extensively in the cells of eucaryotes such as animals, plants and fungi, and they have a wide diversity of functions covering the formation of mitotic apparatus, the development and maintenance of cellular forms, flagellar and ciliary movements, the disposition of intracellular organellas, material transport, hormone secretion, the fluidity of cell membrane, and the like. Especially in nerve cells, microtubules exist as the principal constituent molecules of axons and dendrites, and contribute to material transport by acting as rails for motor proteins. These microtubules are formed by the regular polymerization of the αβ heterodimer of tubulin, and repeatedly show an increase or decrease by polymerization or depolymerization with the progress of the cell cycle. Moreover, the polymerization/depolymerization of these microtubules is also controlled by microtubule-associated proteins (MAPs, τ protein). This control mechanism is primarily based on protein phosphorylation enzymes (kinases) and protein dephosphorylation enzymes (phosphatases) which have those microtubule-associated proteins as substrates. For these reasons, compounds acting on the microtubule system (i.e., microtubule-interfering agents) exhibit various biological activities such as the inhibition of cell divisions, and are hence expected to be widely effective not only as antitumor agents, but also as fungicides, anthelmintics, herbicides and the like.

According to their mode of action, the microtubule-interfering agents are classified into compounds binding to β-tubulin and compounds binding to microtubule-associated proteins. Moreover, the compounds binding to β-tubulin are classified into compounds inhibiting the polymerization of tubulin by binding to β-tubulin, and compounds promoting the abnormal polymerization of tubulin to the contrary. In particular, the compounds inhibiting the polymerization of tubulin suppress cell divisions by inhibiting microtubule formation directly, and are hence expected to exert a selective influence on growing cells. Some such compounds are currently being used as antitumor agents, and others are under development.

On the other hand, ERK-MAP kinases are serine/threonine kinases which occur universally in eucaryotes, and play a major role in the induction of various biological phenomena (e.g., cell growth, nerve cell differentiation, cell movement acceleration, oocyte maturation, etc.) in response to diverse extracellular stimuli. That is, ERK-MAP kinases exactly play a major role in the intracellular signal transfer system where signal transfer reactions are induced by the specific binding of various cell growth/differentiation factors and the like to their respective receptors present on the surfaces of target cells.

In the aforesaid intracellular signal transfer system (i.e., the ERK-MAP kinase cascade), several signal molecules are known to function on the upstream side of the ERK-MAP kinases. The molecules which have been most fully analyzed include Ras, Raf-1, MEK and the like, Specifically, Ras, a GTP-binding protein, is activated in response to external various stimuli. Then, Raf-1 (serine/threonine kinase), which is a sort of MAP kinase kinase kinase (MAPKKK), is activated. Subsequently, Raf-1 phosphorylates/activates MEK1/2 that is a sort of MAP kinase kinase having a unique substrate specificity capable of phosphorylating both serine/threonine residues and tyrosine residues. The activated MEK1/2 converts an inactive form of MAP kinases (ERK1/2) to an active form of MAP kinase by the phosphorylation of T (threonine) and Y (tyrosine) residues in the Thr-Glu-Tyr (TEY) sequence which is present between the subdomains VII and VIII of the kinases. It is believed that the MAP kinases (ERK1/2) activated in the above-described manner migrates from the cytoplasm to the nucleus, where it induces the phosphorylation/activation of several transcription factors and thereby leads to the induction of a final physiological response. As described above, the ERK-MAP kinase cascade is known to participate in the expression of various physiological responses. In particular, it has been clarified that the ERK-MAP kinase cascade plays an essential role in the promotion of cell growth. In other words, it is expected that cell growth can be suppressed by blocking the ERK-MAP kinase cascade.

However, the effectiveness of antitumor agents comprising a combination of a compound acting on microtubules and a compound blocking the ERK-MAP kinase cascade, as described above, has been utterly unknown as yet.

### Disclosure of the Invention

Now, as a result of various investigations on the antitumor effect of microtubule-interfering agents, the present inventors have quite unexpectedly found that, when a microtubule-interfering agent having an antitumor effect is used in combination with an ERK-MAP kinase cascade inhibitor, the antitumor effect of the microtubule-interfering agent is remarkably potentiated.

Thus, according to the present invention, there is provided a combination of a microtubule-interfering agent and an ERK-MAP kinase cascade inhibitor wherein the microtubule-interfering agent and the ERK-MAP kinase cascade inhibitor are administered, either simultaneously or separately with a predetermined interval of time, in order to treat tumors.

According to the present invention, there is also provided an antitumor agent which contains, as an active ingredient, a microtubule-interfering agent for use in combination with an ERK-MAP kinase cascade inhibitor.

According to the present invention, there is also provided an agent for potentiating the antitumor effect of a microtubule-interfering agent which contains an ERK-MAP kinase cascade inhibitor as an active ingredient.

According to the present invention, there is also provided a method for the treatment of a tumor which comprises administering a microtubule-interfering agent and an ERK-MAP kinase cascade inhibitor to a patient, either simultaneously or separately with a predetermined interval of time.

Moreover, according to the present invention, there is provided a method for the use of an ERK-MAP kinase cascade inhibitor for the purpose of potentiating the antitumor effect of a microtubule-interfering agent.

Furthermore, according to the present invention, there is provided a pharmaceutical product comprising a microtubule-interfering agent and/or an ERK-MAP kinase cascade inhibitor, the pharmaceutical product being characterized by having, on or within the packaging material thereof, an indication or document showing the instruction that the microtubule-interfering agent and the ERK-MAP kinase cascade inhibitor should be used in combination.

The term "microtubule-interfering agent" as used herein means any drug that acts on the microtubule system and thereby exhibits an antitumor effect. According to their site of action, they are classified into compounds binding to β-tubulin and compounds binding to microtubule-associated proteins. Moreover, the compounds binding to β-tubulin are classified into compounds inhibiting the polymerization of tubulin by binding to β-tubulin and compounds promoting the abnormal polymerization of tubulin to the contrary. Specifically, the compounds inhibiting the polymerization of tubulin include, for example, dolastatin 10 and compounds analogous thereto, vincristine and compounds analogous thereto, maytansine (THE MERCK INDEX 12th EDITION No. 5800), rhizoxin, phomopsin, ustiloxin and combrestatin. The compounds promoting the polymerization of tubulin include, for example, paclitaxel (THE MERCK INDEX 12th EDITION No. 7117), docetaxel, Taxotere and taxuspine.

On the other hand, the compounds binding to microtubule-associated proteins include, for example, griseofulvin (THE MERCK INDEX 12th EDITION No. 4571) and estramustine (THE MERCK INDEX 12th EDITION No. 3749).

The compound which can be preferably used as microtubule-interfering agents in the present invention are compounds inhibiting the polymerization of tubulin. Among them, dolastatin 10 and compounds analogous thereto, and vincristine and compounds analogous thereto are especially preferred.

Specific examples of these dolastatin 10 and compounds analogous thereto include, for example, the compounds described in references such as Japanese Patent Laid-Open No. 167278/'90, PCT International Publication WO93/03054 Pamphlet, PCT International Publication WO95/09864 Pamphlet, PCT International Publication WO96/33212 Pamphlet, Japanese Patent Laid-Open No. 293795/'94, Japanese Patent Laid-Open No. 70173/'95, Japanese Patent Laid-Open No. 59693/'96, Japanese Patent Laid-Open No. 81493/'96, Japanese Patent Laid-Open No. 119990/'96, Japanese Patent Laid-Open No. 188594/'96, Japanese Patent Laid-Open No. 77791/'97, Published Japanese Translation of PCT International Publication No. 506580/'95 and Published Japanese Translation of PCT International Publication No. 504415/'96.

Among these dolastatin 10 and compounds analogous thereto, the following compound (hereinafter referred to as "TZT-1027") is especially preferred.

N²-(N,N-Dimethyl-L-valyl)-N-[(1S,2R)-2-methoxy-4-[(2S)-2-[(1R,2R)-1-methoxy-2-methyl-3-oxo-3-[(2-phenylethyl)-amino]propyl]-1-pyrrolidinyl]-1-[(S)-1-methylpropyl]-4-oxobutyl]-N-methyl-L-valinamide.

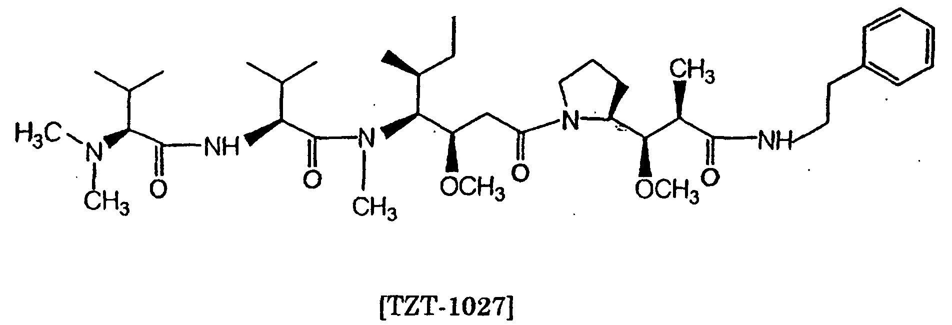

Specific examples of vincristine and compounds analogous thereto include, for example, vincristine (THE MERCK INDEX 12th EDITION No. 10124), vinblastine (THE MERCK INDEX 12th EDITION No. 10119), and vindesine (THE MERCK INDEX 12th EDITION No. 10125).

The term "ERK-MAP kinase cascade inhibitor" as used herein means any compound that acts on a particular molecule in the ERK-MAP kinase cascade and eventually prevents MAP kinase from exhibiting its action effectively. Theoretically, this term comprehends all of the compounds which inhibit the action of MAPKKK and thereby prevent activated MAP kinase kinase from being effectively formed, the compounds which inhibit the action of activated MAP kinase kinase and thereby prevent activated MAP kinase from being effectively formed, and the compounds which inhibit the action of activated MAP kinase.

Specific examples of these ERK-MAP kinase cascade inhibitors include, for example, 2-(2-amino-3-methoxyphenyl)-4H-chromen-4-one (hereinafter referred to as "PD98059"; Proc. Natl. Acad. Sci., Vol. 92, pp. 7686-7689, 1995), 1,4-diamino-2,3-dicyano-1,4-bis(2-aminophenylthio)butadiene (hereinafter referred to as "U1026"; J. BioL Chem., Vol. 273, pp. 18623-18632, 1998), and 2-(2-chloro-4-iodophenylamino)-N-cyclopropylmethoxy-3,4-difluorobenzamide (hereinafter referred to as "PD1843522"; Nature Medicine, Vol. 5, pp. 810-816, 1999).

If necessary, the microtubule-interfering agent or ERK-MAP kinase cascade inhibitor used in the present invention may be reacted with an inorganic or organic acid or an inorganic or organic base to form a pharmaceutically acceptable salt. The inorganic acids which can be used to form a salt include, for example, hydrochloric acid, hydrobromic acid, nitric acid, sulfuric acid and phosphoric acid, and the organic acids which can be used include, for example, acetic acid, propionic acid, oxalic acid, malonic acid, succinic acid, lactic acid, malic acid, tartaric acid, citric acid, benzoic acid and methanesulfonic acid. On the other hand, the inorganic bases which can be used to form a salt include, for example, sodium hydroxide, ammonium hydroxide, calcium carbonate, sodium hydrogen carbonate and calcium hydroxide, and the organic bases which can be used include, for example, methylamine, diethylamine, cyclohexylamine, ethanolamine and morpholine.

The above-described microtubule-interfering agent and ERK-MAP kinase cascade inhibitor may be used in the form of a pharmaceutical preparation having any of dosage forms including solid forms (e.g., tablets, hard capsules, soft capsules, granules, powders, fine subtilaes, pills and troches), semisolid forms (e.g., suppositories and ointments), and liquid forms (e.g., injections, emulsions, suspensions, elixirs, lotions and sprays). The additives which can be used to make such pharmaceutical preparations include, for example, starch, glucose, sucrose, lactose, fructose, maltose, mannitol, sorbitol, cyclodextrin, silicic acid derivatives, methylcellulose, carboxymethylcellulose and its salts, alginates, gelatin, polyvinyl pyrrolidone, calcium carbonate, sodium hydrogen carbonate, magnesium carbonate, talc, magnesium stearate, gum arabic, polyethylene glycol, alkyl p-hydroxybenzoates, cetyl alcohol, syrups, ethanol, propylene glycol, vaseline, carbowax, glycerol, sodium chloride, sodium sulfite, sodium phosphate, citric acid, lactic acid, sodium hydroxide, polylactic acid and polylactic acid-glycolic acid.

The above-described pharmaceutical preparations may be such that both of the active ingredients (i.e., the microtubule-interfering agent and the ERK-MAP kinase cascade inhibitor) are contained in a single pharmaceutical preparation, or such that the microtubule-interfering agent and the ERK-MAP kinase cascade inhibitor are formed into separate pharmaceutical preparations.

In the present invention, so long as the microtubule-interfering agent and the ERK-MAP kinase cascade inhibitor are administered in combination, they may be administered either simultaneously or separately with a predetermined interval of time. Thus, the method of administration may be suitably chosen according to the doctor's diagnosis, the symptoms of the patient, and the like. Where the active ingredients are administered separately, it is generally desirable to administer the ERK-MAP kinase cascade inhibitor in advance.

Moreover, in the present invention, pharmaceutical products comprising the microtubule-interfering agent and/or the ERK-MAP kinase cascade inhibitor are preferably characterized by having, on or within the packaging material thereof, an indication or document showing instructions for the combined use of the microtubule-interfering agent and the ERK-MAP kinase cascade inhibitor.

The contents of the microtubule-interfering agent and the ERK-MAP kinase cascade inhibitor in the pharmaceutical preparations of the present invention may vary according to their dosage form and the like. However, it is generally desirable that the pharmaceutical preparations contain each drug at a concentration of 0.1 to 50% by weight in the case of solid and semisolid forms, and at a concentration of 0.05 to 10% by weight in the case of liquid forms.

The dosages of the microtubule-interfering agent and the ERK-MAP kinase cascade inhibitor may vary widely according to the route of administration, the type and severity of symptoms, the doctor's diagnosis, and the like. However, the generally acceptable effective dose of the microtubule-interfering agent may be in the range of about 0.1 to about 1,000 mg per treatment and preferably about 0.5 to about 500 mg per treatment. The generally acceptable effective dose of the ERK-MAP kinase cascade inhibitor may be in the range of about 0.1 to about 1,000 mg per treatment and preferably about 50 to about 500 mg per treatment.

Nevertheless, it is a matter of course that, depending on the type and severity of symptoms and the doctor's diagnosis as described above, each of the drugs may be administered in a dose less than the lower limit of the aforesaid range or greater than the upper limit thereof. The aforesaid doses of the drugs may be administered at intervals of several hours to one month.

### Examples

The present invention is more specifically explained with reference to the following tests and examples.

### Tests

In the following tests, TZT-1027 or vincristine was used as the microtubule-interfering agent, and PD98059 was used as the ERK-MAP kinase cascade inhibitor.

### 1. Preparation of samples

DMEM medium containing 10% fetal bovine serum was placed in plastic culture dishes having a diameter of 6 cm. Then, WiDr cells (obtained from HSRRB), which were human colon carcinoma cells, were inoculated thereinto so as to give a density of 2.0 x 10⁵ cells/3 mL. These culture dishes were incubated at 37°C for 48 hours. Moreover, 1/1000 volume of PD98059 (with a final concentration of 50 µmol/L) or solvent (DMSO) was added thereto, followed by incubation for 12 hours. Thereafter, 1/1000 volume of TZT-1027 (with a final concentration of 10 nmol/L) or solvent (a lactic acid buffer) was added thereto. Where vincristine was used as the microtubule-interfering agent, 1/1000 volume of vincristine sulfate (with a final concentration of 100 nmol/L) or solvent (DMSO) was added thereto. Twenty-four (24), 48, 72 or 96 hours after the addition of TZT-1027, vincristine or solvent, cells were collected and worked up according to the following procedure. With respect to a solvent control group and a group treated with PD98059 alone, cells were similarly collected and worked up after 96 hours' incubation.

First of all, all of the culture medium was transferred to a centrifuge tube. The remaining cells were suspended by adding 1 mL of a 0.05% trypsin/phosphate-buffered physiological saline (PBS)-EDTA solution to the culture dish and incubating it at 37°C for 5 minutes, and combined with the above culture medium. The centrifuge tube was centrifuged at 1,500 rpm for 5 minutes. Then, the precipitate was resuspended in 1 mL of PBS, transferred to a light-shielding microtube, and centrifuged at 2,500 rpm for 5 minutes. After the precipitate was suspended in 60 µL of PBS, 140 µL of 100% ethanol was added thereto and the resulting suspension was stirred and allowed to stand at -20°C overnight or longer. Moreover, the suspension was centrifuged at 2,500 rpm for 5 minutes, and 100 µL of a phosphate-citric acid buffer (0.2M Na₂PO₄ : 0.1M citric acid = 24:1) was added to the precipitate, followed by stirring (at 500 rpm) at room temperature for 30 minutes. After suspension was centrifuged at 2,500 rpm for 5 minutes, 100 µL of PBS and 1 µL of an RNase solution (10 mg/mL (PBS); obtained from SIGMA) was added to the precipitate, followed by incubation at 37°C for 30 minutes. Thereafter, 880 µL of PBS and 20 µL of a propidium iodide (hereinafter referred to as "PI") solution (1 mg/mL (PBS); obtained from Wako Pure Chemical Industries Ltd.) were added thereto and reacted at room temperature for 30 minutes with stirring. The resulting reaction mixture was allowed to stand in the dark under cooling with ice until it was used for measurement.

### 2. Cell death rate

The cell death rate was measured and analyzed by means of an EPICS XL system (manufactured by COULTER). Prior to analysis, the EPICS XL system was checked by means of DNAcheck (manufactured by COULTER).

Next, a protocol used for calculating the cell death rate by using the DNA content as a parameter was constructed. In this protocol, FS (forward scatter), SS (side scatter) and FL3 (PI fluorescence) were selected as parameters, and the number of analyzed cells was 30,000. Furthermore, by measuring a control sample of cells to be actually measured, the sensitivity of the detector and regions were preset with consideration for each parameter. Measurements were made by pipetting a sample at several times, transferring it into an exclusive tube, and setting it in the main unit of the EPICS XL system. The cell death rate was calculated by use of histograms in the protocol, which were constructed by removing doublets and triplets from data incorporating dead cells. The results of the measurements are shown in Table 1 below.

**Table 1**

| Microtubule interfering-agent | ERK-MAP kinase cascade inhibitor | Time | Cell death rate (%) |
|---|---|---|---|
| None (lactic acid buffer) | None (0.1% DMSO) | 96 | 3.0 |
| None (lactic acid buffer) | PD98059 (50 µmol/L) | 96 | 8.9 |
| TZT-1027 (10 nmol/L) | None (treated with the solvent alone) | 24 | 3.8 |
| | | 48 | 9.2 |
| | | 96 | 23.3 |
| TZT-1027 (10 nmol/L) | PD98059 (50 µmol/L) | 24 | 10.5 |
| | | 48 | 35.9 |
| | | 96 | 48.1 |
| Vincristine (100 nmol/L) | None (treated with the solvent alone) | 24 | 4.5 |
| | | 48 | 8.4 |
| | | 72 | 23.3 |
| Vincristine (100 nmol/L) | PD98059 (50 µmol/L) | 24 | 12.1 |
| | | 48 | 37.4 |
| | | 72 | 58.7 |

### Example 1

| Injection: | | |
|---|---|---|
| | | mg/ampule |
| Active component | TZT-1027 | 0.2 |
| Tonicity agent | Sodium chloride | q.s. |
| pH regulator | Lactic acid | q.s. |
| pH regulator | Sodium hydroxide | q.s. |
| Solvent | Water for injection | q.s. |
| | | 1 mL |

Sodium chloride and lactic acid are dissolved in water for injection, and the active ingredient is dissolved in the resulting solution. After the pH of the solution is adjusted to 4-5 with sodium hydroxide, water for injection is added thereto until the prescribed volume is reached.

| Tablets: | |
|---|---|
| | mg/tablet |
| PD98059 | 20.0 |
| Starch | 5.0 |
| Lactose | 132.0 |
| Carboxymethylcellulose calcium | 10.0 |
| Talc | 1.0 |
| Magnesium stearate | 2.0 |
| | 170.0 |

PD98059 is pulverized to a particle size of 70 microns or less. Then, starch, lactose and carboxymethylcellulose calcium are added thereto and thoroughly mixed therewith. After the addition of 10% starch paste, the above powder mixture is agitated and blended to prepare granules. After drying, these granules are adjusted to a particle diameter of about 1,000 microns, and mixed with talc and magnesium stearate. The resulting mixture is formed into tablets.

## Claims

1. A combination of a microtubule-interfering agent and an ERK-MAP kinase cascade inhibitor wherein the microtubule-interfering agent and the ERK-MAP kinase cascade inhibitor are administered, either simultaneously or separately with a predetermined interval of time, in order to treat a tumor.

2. A combination as claimed in claim 1 wherein the microtubule-interfering agent is a tubulin polymerization inhibitor.

3. A combination as claimed in claim 2 wherein the tubulin polymerization inhibitor is dolastatin 10 or a compound analogous thereto, or vincristine or a compound analogous thereto.

4. A combination as claimed in claim 1 wherein the ERK-MAP kinase cascade inhibitor is a MAP kinase inhibitor, a MAP kinase kinase inhibitor or a MAP kinase kinase kinase inhibitor.

5. An antitumor agent which contains, as an active ingredient, a microtubule-interfering agent for use in combination with an ERK-MAP kinase cascade inhibitor.

6. An agent for potentiating the antitumor effect of a microtubule-interfering agent which contains an ERK-MAP kinase cascade inhibitor as an active ingredient.

7. A method for the treatment of a tumor which comprises administering a microtubule-interfering agent and an ERK-MAP kinase cascade inhibitor to a patient, either simultaneously or separately with a predetermined interval of time.

8. The use of an ERK-MAP kinase cascade inhibitor for the purpose of potentiating the antitumor effect of a microtubule-interfering agent.

9. A pharmaceutical product comprising a microtubule-interfering agent and/or an ERK-MAP kinase cascade inhibitor, the pharmaceutical product being **characterized by** having, on or within the packaging material thereof an indication or document showing the instruction that the microtubule-interfering agent and the ERK-MAP kinase cascade inhibitor should be used in combination.
